# EUROPEAN PATENT APPLICATION

(11) **EP 4 092 105 A1**
(43) Date of publication of application: **23.11.2022**
(21) Application number: 21741902.7
(22) Date of filing: 13.01.2021
(51) Int. Cl.: C12N 5/02, C12N 5/074, C12N 5/10

(54) **HIGH-DENSITY CELL CULTURE METHOD**

(30) Priority: 14.01.2020 JP 2020003959
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); HIGUCHI, Takuya, Kawasaki-shi, Kanagawa 210-8681 (JP); FUROMITSU, Shumpei, Kawasaki-shi, Kanagawa 210-8681 (JP); NISHIYAMA, Megumi, Kawasaki-shi, Kanagawa 210-8681 (JP)
(74) Representative: Hoffmann Eitle
(86) International application number: PCT/JP2021/000746
(87) International publication number: WO 2021/145321

(57) **Abstract**

A method for culturing cells, including adding choline at a specific concentration to a medium is provided by the present invention.

## Description

### [Technical Field]

The present invention relates to a high density culture method for cells. More particularly, the present invention relates to a high density culture method for cells, including addition of choline.

### [Background Art]

Regenerative medicine technology using cells has been receiving high attention in recent years as one of the means capable of treating various diseases and damages that were difficult to treat before. Since regenerative medicine requires a large amount of cells, the development of a method for efficiently culturing cells has been actively conducted. For example, Patent Literature 1 reports a method for culturing stem cells, including treating stem cells with a ROCK inhibitor in a medium. In addition, the present inventors also reported in 2018 a high density culture method of animal cells, including adding glucose and/or specific amino acids to a medium, and the like (Patent Literature 2).

### [Citation List]

### [Patent Literature]

[PTL 1]
   JP-A- 2008-099662
[PTL 2]
   Japanese Patent Application No. 2018-184352

### [Summary of Invention]

### [Technical Problem]

The method described in Patent Literature 2 is one of the very superior methods for high density culture. This time, the present inventors reconsidered the method and attempted to develop a more preferable high density culture method.

### [Solution to Problem]

The present inventors have conducted intensive studies of the above-mentioned problems and found that cell proliferation can be extremely easily promoted by further adding choline, from among various medium components, during high density culture of the cells. Based on such findings, they have conducted further studies and completed the present invention.

Accordingly, the present invention provides the following.
[1] A method for culturing cells, comprising adding choline to a medium at 0.01 to 1000000 mg/L/day.
[2] The method of [1], wherein the cell culture is suspension culture.
[3] The method of [1] or [2], wherein the method is for use in high density culture.
[4] The method of [3], wherein cells to be cultured have a cell density in a medium of not less than 6.0×10⁵ cells/mL.
[5] The method of any of [1] to [4], wherein the cell is a pluripotent stem cell, an adult stem cell, or a progenitor cell.
[6] The method of [5], wherein the pluripotent stem cell is an iPS cell.
[7] The method of any of [1] to [4], wherein the cell is derived from an iPS cell.
[8] A cell culture medium composition comprising choline at not less than 15 mg/L.
[9] The medium composition of [8], wherein the composition is for suspension culture.
[10] The medium composition of [8] or [9], wherein the composition is for high density culture.
[11] The medium composition of [10], wherein cells to be subjected to suspension culture have a cell density of not less than 6.0×10⁵ cells/mL.
[12] The medium composition of any of [8] to [11], wherein the cell is a pluripotent stem cell, an adult stem cell, or a progenitor cell.
[13] The medium composition of [12], wherein the pluripotent stem cell is an iPS cell.
[14] The medium composition of any of [8] to [13], wherein the cell is derived from an iPS cell.

### [Advantageous Effects of Invention]

According to the present invention, cell proliferation can be promoted easily under high density culture.

### [Brief Description of Drawings]

[Fig. 1]

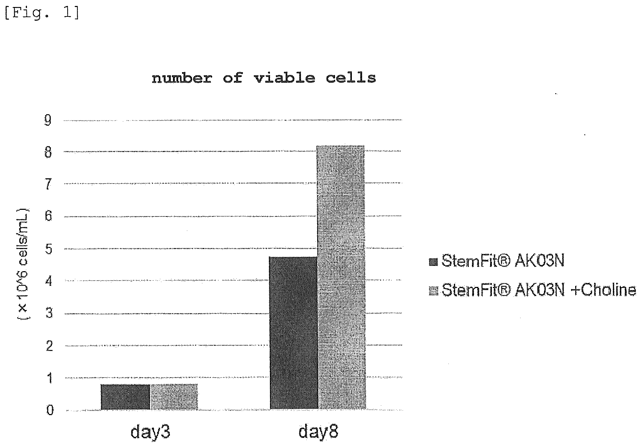

Fig. 1 shows the influence of the choline concentration on the proliferation of iPS cells (n=1).

### [Description of Embodiments]

The present invention is explained in detail in the following.

### Definition

In the present specification, the "suspension culture" refers to a cell culture method performed in a state where cells do not adhere to the culture container. In the present invention, the suspension culture may or may not be accompanied by pressure from the outside or vibration on the liquid medium, or shaking or rotation operation in the liquid medium.

In the present specification, the "high density culture" refers to a culture at a high cell density compared to the cell density expected in general cell culture. The criteria for high density may vary depending on the culture method (contact culture/suspension culture, etc.), type of pluripotent stem cells, and the like. For example, in the case of suspension culture of iPS cells, culture at a density of not less than 6×10⁵ cells/mL is defined as the high density culture in the present specification.

In the present specification, the "pluripotent stem cell" means a cell capable of differentiating into any tissue or cell constituting living organisms. Examples of the pluripotent stem cell include, but are not limited to, embryonic stem cell (ES cell), embryonic germ cell (EG cell), induced pluripotent stem cell (iPS cell), and the like.

In the present specification, the "adult stem cell (also called somatic stem cell)" means a cell that has the ability to differentiate into a cell constituting a specific tissue (organ). Examples of the adult stem cell include, but are not limited to, hematopoietic stem cell, neural stem cell, germ stem cell, intestinal stem cell, epidermis stem cell, mesenchymal stem cell, and the like.

In the present specification, the "progenitor cell" means a cell in the process of differentiating from the aforementioned pluripotent stem cell or adult stem cell into a specific somatic cell or reproductive cell.

### 1. cell culture method

The present invention provides a method for culturing cells, including adding choline to a medium at 0.01 to 1000000 mg/L/day (hereinafter sometimes referred to as "the method of the present invention").

The choline to be used in the method of the present invention is not particularly limited as long as it is of the grade used for cell culture, and a commercially available product may be used. Preferred choline includes choline chloride, choline bitartrate, choline bicarbonate, choline phosphate, choline dihydrogen citrate and the like, with preference given to choline chloride.

In the method of the present invention, the amount of choline (content after conversion to free form) to be added to the medium is generally 0.01 to 1000000 mg/L/day, preferably 0.1 to 1000 mg/L/day, more preferably 1 to 100 mg/L/day (for example,
[1] 1 to 100 mg/L/day, 10 to 100 mg/L/day, 20 to 100 mg/L/day, 30 to 100 mg/L/day, 40 to 100 mg/L/day, 50 to 100 mg/L/day, 60 to 100 mg/L/day, 70 to 100 mg/L/day, 80 to 100 mg/L/day, 90 to 100 mg/L/day;
[2] 1 to 90 mg/L/day, 10 to 90 mg/L/day, 20 to 90 mg/L/day, 30 to 90 mg/L/day, 40 to 90 mg/L/day, 50 to 90 mg/L/day, 60 to 90 mg/L/day, 70 to 90 mg/L/day, 80 to 90 mg/L/day;
[3] 1 to 80 mg/L/day, 10 to 80 mg/L/day, 20 to 80 mg/L/day, 30 to 80 mg/L/day, 40 to 80 mg/L/day, 50 to 80 mg/L/day, 60 to 80 mg/L/day, 70 to 80 mg/L/day;
[4] 1 to 70 mg/L/day, 10 to 70 mg/L/day, 20 to 70 mg/L/day, 30 to 70 mg/L/day, 40 to 70 mg/L/day, 50 to 70 mg/L/day, 60 to 70 mg/L/day;
[5] 1 to 60 mg/L/day, 10 to 60 mg/L/day, 20 to 60 mg/L/day, 30 to 60 mg/L/day, 40 to 60 mg/L/day, 50 to 60 mg/L/day;
[6] 1 to 50 mg/L/day, 10 to 50 mg/L/day, 20 to 50 mg/L/day, 30 to 50 mg/L/day, 40 to 50 mg/L/day;
[7] 1 to 40 mg/L/day, 10 to 40 mg/L/day, 20 to 40 mg/L/day, 30 to 40 mg/L/day;
[8] 1 to 30 mg/L/day, 10 to 30 mg/L/day, 20 to 30 mg/L/day;
[9] 1 to 20 mg/L/day, 10 to 20 mg/L/day).

The medium to be used in the method of the present invention is not particularly limited, and a medium suitable for the type of cells to be cultured can be used. Such medium may be prepared by a method known per se or a commercially available product may also be used.

Examples of the commercially available medium include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the medium particularly for stem cell culture include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

In addition, components preferable for cell proliferation can be further added to these media. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estra diol, progesterone, glucagon, insulin, and the like; and the like. Appropriate components can be selected and used according to the type of the cells to be cultured.

In one preferred embodiment, D-glucose and five kinds of amino acids (tryptophan, serine, cysteine (or cystine), methionine, arginine) may be further added to the medium.

Glucose (or a salt thereof) can be added to the medium of the present invention such that the converted glucose concentration is generally 0.1 g/L/day to 900 g/L/day, preferably 1 g/L/day to 200 g/L/day, more preferably 1 g/L/day to 20 g/L/day.

In addition, five kinds of amino acids (tryptophan, serine, cysteine (cystine), methionine, and arginine) can be added to the medium of the present invention such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

The cell type to which the method of the present invention can be applied is not particularly limited. Examples of such cell type include germ cells such as spermatozoon, ovum, and the like, somatic cells constituting the living body, stem cells (pluripotent stem cell, etc.), precursor cells, cancer cells separated from the living body, cells (cell lines) that are separated from the living body, acquire immortalizing ability, and are stably maintained ex-vivo, cells that are separated from the living body and subjected to artificial gene modification, cells that are separated from the living body and subjected to artificial nuclear exchange, and the like. Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermis cell, endothelial cell, vascular endothelial cell, hepatocyte, chondrocyte, cumulus cell, neuronal cells, glial cell, neuron, oligodendrocyte, micro glia, astrocyte, heart cell, esophageal cell, muscle cells (e.g., smooth myocyte or skeleton myocyte), pancreas beta cell, melanocyte, hematopoietic progenitor cell (e.g., CD34 positive cell derived from cord blood), mononuclear cell, and the like. The somatic cell includes, for example, cells taken from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood (including cord blood), bone marrow, heart, eye, brain, neural tissue, and the like.

Stem cell is a cell that has the ability to replicate itself and the ability to differentiate into other multi-lineage cells. Examples thereof include, but are not limited to, embryonic stem cell (ES cell), embryonal carcinoma cell, embryonic germ cell, induced pluripotent stem cell (iPS cell), neural stem cell, hematopoietic stem cell, mesenchymal stem cell, hepatic stem cell, pancreatic stem cell, muscle stem cell, germ stem cell, intestinal stem cell, cancer stem cell, hair follicle stem cell, and the like.

A cell line is a cell that has acquired infinite proliferation potency through artificial manipulation outside the body. Examples thereof include, but are not limited to, CHO (Chinese hamster ovary cell line), HCT116, Huh7, HEK293 (human fetal kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trade mark), Vero, and the like.

In one embodiment, the cell may be, but is not limited to, a pluripotent stem cell, an adult stem cell, or a progenitor cell.

In one embodiment, the cell may be a pluripotent stem cell. In this case, it is also preferable to further add bFGF to the medium.

In one embodiment, the pluripotent stem cell may be an embryonic stem cell (ES cell) or an iPS cell, preferably an iPS cell. The adult stem cell may be, but is not limited to, a hematopoietic stem cell, a neural stem cell, a germ stem cell, an intestinal stem cell, an epidermis stem cell, or a mesenchymal stem cell. The origin of the pluripotent stem cell, adult stem cell, and progenitor cell is also not particularly limited, but those derived from mammals are preferred, and those derived from human are more preferred.

In one embodiment, the cell may be a cell derived from an iPS cell. The cell derived from an iPS cell means a cell in the process of differentiating or differentiated from an iPS cell by a method known per se.

In the method of the present invention, the culture conditions are not particularly limited, and a method known per se may be selected according to the cell type, cell density, culture method (adhesion culture/suspension culture, etc.), and the like. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume.

### 2. cell culture medium composition

The present invention provides a medium composition for cell culture, containing choline at not less than 15 mg/L (hereinafter sometimes referred to as "the medium composition of the present invention").

The medium composition of the present invention is characterized by addition of high concentration of choline to a general basal medium for cells.

At the time of filing the present application, the amount of choline contained in the medium for culturing cells is about 14 mg/L at most. This is because the effect on cell proliferation and/or undifferentiation is considered to be constant even when choline is added at a higher concentration. The lower limit of the concentration of choline contained in the medium composition of the present invention may be generally 15 mg/L, preferably 16 mg/L, more preferably 17 mg/L, further preferably 18 mg/L, particularly preferably 20 mg/L, or may be higher. The upper limit is not particularly set, and may be generally 1000000 mg/L, preferably 10000 mg/L, more preferably 1000 mg/L, further preferably 100 mg/L, particularly preferably 50 mg/L, from the aspects of the cost and the like. In one embodiment, the concentration of choline in the medium composition of the present invention may be generally 15 mg/L to 1000000 mg/L, preferably 16 mg/L to 100000 mg/L, more preferably 17 mg/L to 10000 mg/L, further preferably 18 mg/L to 100 mg/L, particularly preferably 20 mg/L to 50 mg/L.

The medium for cells which is used in preparing the medium composition of the present invention may be prepared by a method known per se according to the cells to be cultured, or a commercially available product.

The medium that can be used for producing the medium composition of the present invention, the component that can be further added to the medium composition of the present invention, and the cell type to which the medium composition of the present invention is applicable are the same as those explained in "1. cell culture method".

The present invention is explained in more detail in the following Examples; however, the present invention is not limited by these Examples.

### [Example]

### Example

In the following Example, the proliferation effect of induced pluripotent stem cells (iPS cells) by choline were evaluated. As the iPS cell, 1210B2 strain purchased from iPS Academia Japan was used. In addition, as a medium for iPS cells, a commercially available StemFit AK03N (Ajinomoto Co., Inc.) was used.

### [Example 1] Effect of iPS cell proliferation promotion by enriching choline using suspension culture system

Using a 30 mL single use bioreactor for iPS cells (ABLE: BWV-S03A), iPS cell 1210B2 strain was seeded in StemFit AK03N+10 µM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit AK03N or StemFit AK03N+10 mg/L choline chloride (FUJIFILM Wako Pure Chemical Corporation). The cells were transferred to a micro bioreactor ambr15 (sartorius:001-0881), and stirring culture was continued under the conditions of 37°C, pH=7.2, dissolved oxygen concentration=20%, stirring speed=300 rpm. 70% of the medium was exchanged with StemFit AK03N or StemFit AK03N+10 mg/L choline chloride once per day, and 40 mg/L/day Trp (Ajinomoto Co., Inc.), 40 mg/L/day Ser (Ajinomoto Co., Inc.), 40 mg/L/day Cys hydrochloride (Japan protein), 40 mg/L/day Met (Ajinomoto Co., Inc.), 160 mg/L/day Arg (Ajinomoto Co., Inc.), 4 g/L/day D-glucose (Nacalai Tesque:16806-25) were further added to both groups. On the 8th day of culture, the number of viable cells was measured using Vi-CELL^{™} XR (Beckman Coulter), a live/dead cell autoanalyzer.

The verification results of the influence of choline concentration on the proliferation of iPS cells in n=1 are shown in Fig. 1. The results showing a cell proliferation promoting effect could be obtained by the addition of choline.

### [Industrial Applicability]

According to the present invention, extremely efficient high density culture can be performed by a highly simple and inexpensive method.

This application is based on a patent application No. 2020-003959 filed in Japan (filing date: January 14, 2020), the contents of which are incorporated in full herein.

## Claims

1. A method for culturing cells, comprising adding choline to a medium at 0.01 to 1000000 mg/L/day.

2. The method according to claim 1, wherein the cell culture is suspension culture.

3. The method according to claim 1 or 2, wherein the method is for use in high density culture.

4. The method according to claim 3, wherein cells to be cultured have a cell density in a medium of not less than 6.0×10⁵ cells/mL.

5. The method according to any one of claims 1 to 4, wherein the cell is a pluripotent stem cell, an adult stem cell, or a progenitor cell.

6. The method according to claim 5, wherein the pluripotent stem cell is an iPS cell.

7. The method according to any one of claims 1 to 4, wherein the cell is derived from an iPS cell.

8. A cell culture medium composition comprising choline at not less than 15 mg/L.

9. The medium composition according to claim 8, wherein the composition is for suspension culture.

10. The medium composition according to claim 8 or 9, wherein the composition is for high density culture.

11. The medium composition according to claim 10, wherein cells to be subjected to suspension culture have a cell density of not less than 6.0×10⁵ cells/mL.

12. The medium composition according to any one of claims 8 to 11, wherein the cell is a pluripotent stem cell, an adult stem cell, or a progenitor cell.

13. The medium composition according to claim 12, wherein the pluripotent stem cell is an iPS cell.

14. The medium composition according to any one of claims 8 to 13, wherein the cell is derived from an iPS cell.
